**Europäisches Patentamt**

**European Patent Office**

**Office européen des brevets**

(19)

(11) Veröffentlichungsnummer : **0 467 026 B1**

## (12) EUROPÄISCHE PATENTSCHRIFT

(45) Veröffentlichungstag der Patentschrift :
**03.03.93 Patentblatt 93/09**

(51) Int. Cl.$^5$ : **A61K 7/13**

(21) Anmeldenummer : **91106839.3**

(22) Anmeldetag : **26.04.91**

---

(54) **Mittel zur Färbung von Haaren und Anwendung desselben.**

---

Die Akte enthält technische Angaben, die nach dem Eingang der Anmeldung eingereicht wurden und die nicht in dieser Patentschrift enthalten sind.

(30) Priorität : **17.07.90 DE 4022724**
**24.12.90 DE 4041741**

(43) Veröffentlichungstag der Anmeldung :
**22.01.92 Patentblatt 92/04**

(45) Bekanntmachung des Hinweises auf die Patenterteilung :
**03.03.93 Patentblatt 93/09**

(84) Benannte Vertragsstaaten :
**AT BE CH DE DK ES FR GB IT LI LU NL**

(56) Entgegenhaltungen :
**DE-A- 2 523 629**
**DE-A- 2 554 456**

(73) Patentinhaber : **GOLDWELL AKTIENGESELLSCHAFT**
**Zerninstrasse 10-18**
**W-6100 Darmstadt 13 (DE)**

(72) Erfinder : **Kufner, Frank**
**Stockhausenweg 43**
**W-6100 Darmstadt 13 (DE)**
Erfinder : **Lorenz, Heribert**
**Ober-Ramstädter Strasse 22**
**W-6101 Gross-Bieberau (DE)**
Erfinder : **Tennigkeit, Jürgen, Dr.-Ing.**
**Felsbergstrasse 51**
**W-6104 Seeheim 2 (Balkhausen) (DE)**

---

## Beschreibung

Die Erfindung betrifft ein Mittel für die oxidative Färbung von menschlichen oder tierischen Haaren, deren Färbewirkung auf der Reaktion bestimmter Entwickler- und Kupplersubstanzen in Gegenwart von Wasserstoffperoxid bzw. Sauerstoff beruht, sowie die Anwendung dieses Mittels.

Auf dem Gebiet der Haarfärbung werden überwiegend Oxidationsfarbstoffe angewandt, bei denen die Färbung durch Reaktion bestimmter Entwicklersubstanzen mit bestimmten Kupplersubstanzen in Gegenwart eines geeigneten Oxidationsmittels erfolgt. Dabei werden an die Oxidationsfarbstoffe, die zur Färbung menschlicher Haare Verwendung finden, verschiedene Anforderungen gestellt:

So müssen bei der oxidativen Kupplung zwischen den jeweiligen Entwickler- und Kupplersubstanzen die gewünschten Farbnuancen in ausreichender Intensität und Beständigkeit gebildet werden, d. h., die Färbung muß eine gute Licht-, Dauerwell-, Säure- und Reibechtheit haben. Außerdem soll die Färbung auch eine hinreichende Haltbarkeit haben und etwa vier bis sechs Wochen stabil sein. Sie soll außerdem zu möglichst geringer Schädigung der Haarstruktur führen; darüber hinaus müssen die verwendeten Farbstoffkomponenten in toxikologischer und dermatologischer Hinsicht unbedenklich sein.

Bei den bisher eingeführten Haarfärbemitteln werden die geschilderten Forderungen teilweise nicht in vollem Umfange erreicht, z. B. werden bei empfindlichen Personen Sensibilisierungen und als Folge hieraus Allergien durch die verwendeten Farbstoffkomponenten beobachtet. Insbesondere bei alkalisch eingestellten Oxidations-Farbstoffen wird außerdem, insbesondere bei wiederholter Einwirkung, z. B. bei mehrfachem Nachfärben, eine Schädigung der Struktur des Haares beobachtet.

Um solche Strukturschädigungen zu verringern, wurden auch bereits sauer eingestellte Haarfärbemittel entwickelt, wobei die ursprünglich beobachtete unzureichende Färbewirkung im sauren Bereich durch Beifügung geringer Mengen von Katalysatorstoffen, z. B. Mangandioxid, verbessert wurde (DE-PS 35 30 270). Aber auch solche sauer eingestellten Haarfärbemittel weisen noch nicht immer alle geforderten Eigenschaften auf.

Die Erfindung geht daher von der Aufgabenstellung aus, ein Mittel zur oxidativen Haarfärbung zu entwickeln, in welchem die geschilderten Anforderungen in noch höherem Maße erfüllt bzw. die dargelegten Nachteile vermieden oder doch zumindest weitgehend vermindert werden.

Ausgehend von einem Mittel der eingangs erwähnten Art wird diese Aufgabe erfindungsgemäß dadurch gelöst, daß es als Entwicklersubstanz alleine oder in Mischung mit anderen Entwicklersubstanzen 6-OH-2,4,5-Triaminopyrimidin (4-OH-2,5,6-Triaminopyrimidin) und/oder 5-OH-2, 4, 6-Triaminopyrimidin enthält.

Eingehende Untersuchungen haben gezeigt, daß sich unter Verwendung bzw. Mitverwendung dieser Hydroxytriaminopyrimidine als Entwicklersubstanzen Rezepturen für Haarfärbepräparate entwickeln lassen, welche die gestellten Anforderungen in hohem Maße erfüllen.

Aus der DE-A 25 54 456 ist bereits die Verwendung von 2-Hydroxy-4,5,6-triaminopyrimidin als Entwicklersubstanz in Oxidations-Haarfärbemitteln bekannt. Wie dem Fachmann jedoch bekannt ist, läßt sich aus dem Verhalten von in Oxidations-Haarfarben bereits bekannten Inhaltsstoffen nicht auf die mit strukturell ähnlichen Substanzen erzielbaren Farbergebnissen schließen, was durch Vergleichsversuche auch im vorliegenden Fall bestätigt wurde.

Als Kupplersubstanz kommen für das erfindungsgemäße Mittel vorzugsweise Resorcin, 4-Chlorresorcin, m-Aminophenol, m-Phenylendiamin, α-Naphthol, p-Amino-4-hydroxyethylaminoanisol, o-Aminophenol, o-Chlor-p-phenylendiamin, 1,7-Dihydroxynaphthalin, 3-Dimethylaminophenol oder Gemische davon in Frage.

Als zweckmäßig hat es sich dabei erwiesen, wenn die Entwicklersubstanz in einer Konzentration von etwa 0,05 Mol mit etwa der gleichen Menge Kupplersubstanz im Haarfärbemittel enthalten ist.

Der bevorzugte Bereich für die erfindungsgemäßen Entwicklersubstanzen liegt zwischen 0,01 und 5, vorzugsweise 0,1 und 4, insbesondere 0,5 und 3 Gew.-% des Haarfärbemittels (ohne Oxidationsmittel). Der bevorzugte Anteil der Kupplersubstanzen liegt etwa im gleichen Bereich.

Als weitere Entwicklersubstanzen im Gemisch mit den erfindungsgemäßen sind beispielsweise p-Phenylendiamin, p-Toluylendiamin, 4-Aminophenol, Tetraaminopyrimidine,etc. geeignet.

Versuche haben außerdem gezeigt, daß das erfindungsgemäße Haarfärbemittel sowohl im alkalischen als auch im sauren Bereich wirksam ist, d. h., daß seine Rezeptur auf einen pH-Wert $\geqq 7$ ebenso wie $\leqq 7$ eingestellt werden kann.

Bei der Anwendung des erfindungsgemäßen Mittels kann dieses in an sich bekannter Weise vor der Aufbringung mit einem Oxidationsmittel, vorzugsweise Wasserstoffperoxid, vermischt werden.

Es hat sich gezeigt, daß die Reaktion der Entwickler- mit den Kupplersubstanz(en) in dem auf das zu färbende Haar aufgetragenen Färbemittel auch mittels Luftsauerstoff durchgeführt werden kann.

Die erfindungsgemäßen Haarfärbemittel werden in üblicher Weise in eine für die Anwendung geeignete Form, d. h. als Cremes, Emulsionen, Gele oder auch Lösungen aufbereitet, wobei die üblichen kosmetischen Zusätze, wie Parfümöle, Komplexbildner, Netzmittel, Emulgatoren, Verdickungsmittel und Antioxydantien enthalten sein können.

Anhand der nachstehenden aufgeführten Beispiele wird das erfindungsgemäße Haarfärbemittel näher erläutert.

Eine Übersicht über die in Haarfärbemitteln üblicherweise zum Einsatz gelangenden Kuppler, Nuanceure, Hilfsstoffe, Grundrezepturen etc. findet sich in der Monographie von K. Schrader, Grundlagen und Rezepturen der Kosmetika, 2. Auflage (1989, Hüthig Verlag), S. 782 - 815, auf die Bezug genommen wird.

Beispiel 1

| | |
|---|---|
| Cetylstearylalkohol | 10,00 g |
| Kokosfettsäuremonoethanolamid | 2,00 g |
| Stearinsäuremonoethanolamid | 2,00 g |
| Stearinsäurediethanolamid | 1,00 g |
| Ölsäure | 3,00 g |
| | |
| 4-Hydroxy-2,5,6-triaminopyrimidin | 1,50 g |
| 1-Naphthol | 0,60 g |
| 2-Methylresorcin | 0,40 g |
| 2-Amino-4-hydroxyethylaminoanisol | 0,40 g |
| 3-Dimethylaminophenol | 0,10 g |
| | |
| Ammoniak 25 % | 12,00 g |
| Natriumsulfit | 0,50 g |
| Natriumlaurylsulfat | 0,20 g |
| Ethylendiaminotetraessigsäure | 0,10 g |
| Parfüm | 0,20 g |
| Enth. Wasser | auf  100,00 g |

Das nach dieser Rezeptur erhaltene Haarfärbemittel wird zu gleichen Teilen mit einer 6prozentigen $H_2O_2$-Lösung vermischt, wobei ein pH-Wert von 9,5 erreicht wird, und auf das Haar aufgetragen. Nach 30 Min. Einwirkungszeit erhält man einen kräftigen Violett-Ton.

EP 0 467 026 B1

Beispiel 2

| | |
|---|---|
| Cetylstearylalkohol | 10,00 g |
| Kokosfettsäuremonoethanolamid | 2,00 g |
| Stearinsäuremonoethanolamid | 2,00 g |
| Stearinsäurediethanolamid | 1,00 g |
| Ölsäure | 3,00 g |
| 4-Hydroxy-2,5,6-triaminopyrimidin | 0,85 g |
| 2-Methylresorcin | 0,10 g |
| 1-Naphthol | 0,10 g |
| 2-Amino-4-hydroxyethylaminoanisol | 0,25 g |
| 3-Dimethylaminophenol | 0,40 g |
| Natriumhydroxid | 0,30 g |
| Natriumsulfit | 0,25 g |
| Mangandioxid | 0,60 g |
| Natriumlaurylsulfat | 0,50 g |
| Ethylendiaminotetraessigsäure | 0,20 g |
| Parfüm | 0,20 g |
| Enth. Wasser | auf 100,00 g |

Das aus dieser Rezeptur resultierende Haarfärbemittel ist leicht sauer (pH 6,8), nachdem es für die Anwendung im Verhältnis 1 : 2 mit einer 6prozentigen $H_2O_2$-Lösung vermischt wird. Nach 30-minütiger Einwirkungszeit erhält man einen intensiven Violett-Ton im Dunkelblondbereich.

Beispiel 3

| | |
|---|---|
| Oleylalkohol mit 5 Mol EO | 1,50 g |
| Stearinsäurediethanolamid | 3,00 g |
| 4-Hydroxy-2,5,6-triaminopyrimidin | 0,50 g |
| o-Chlor-p-phenylendiamin | 0,50 g |

4

| Ethylendiaminotetraessigsäure | 0,10 g |
| Ammoniak 25 % | 0,50 g |
| Cetyltrimethylammoniumchlorid | 1,00 g |
| Enthärtetes Wasser | auf 100,00 g |

Das Haarfärbemittel nach dieser Rezeptur weist einen pH-Wert von 8,5 auf und wird aus einer Aerosol-Verpackung mit einem Propan/-Butan-Gemisch als Treibmittel ausgeschäumt und auf das zu färbende Haar aufgebracht.

Nach einer Einwirkungszeit von 30 Min. entsteht ein hellblonder Goldton. In diesem Fall wird vor der Anwendung nicht mit einem Oxidationsmittel gemischt, sondern die Reaktion erfolgt durch Oxidation mit Luft-Sauerstoff.

Nachstehend wird noch in tabellarischer Zusammenstellung das bei der Verwendung unterschiedlicher Kupplersubstanzen in Verbindung mit den erfindungsgemäßen Entwicklern (Hydroxytriaminopyrimidin) tendenziell zu erwartende Färbeergebnis angegeben, wobei grundsätzlich unterschieden wird zwischen Aufbereitungen, bei denen dem Färbemittel vor der Anwendung ein Oxidationsmittel zugemischt wird und denen, wo lediglich eine Luft-Oxidation erfolgt. Dabei wird dann jeweils weiter zwischen Rezepturen für die betrachteten Substanzen unterschieden, die nach der fertigen Aufbereitung entweder alkalisch oder sauer eingestellt sind.

Die in der Tabelle aufgeführten Entwicklersubstanzen wurden in einer Konzentration von 0,05 Mol mit der gleichen Menge (0,05 Mol) Kupplersubstanz zur Reaktion gebracht.

Die Ausfärbungen wurden auf standardisiertem, zu 60 % ergrautem Menschenhaar, Büffelhaar und einem Woll-Läppchen ausgeführt, wobei die Färbedauer in allen Fällen 30 Min. bei Raumtemperatur betrug.

Nach Beendigung des Färbeprozesses wurden die Muster zunächst ausgespült, mit einem Haarwaschmittel ausgewaschen und anschließend getrocknet.

| Beispiel | Entwickler | Kuppler | Farbton des gefärbten Haares nach der Oxidation mit 6%iger Wasserstoffperoxidlösung | | Farbton des gefärbten Haares nach der Luftoxidation | |
|---|---|---|---|---|---|---|
| | | | alkalisch | sauer | alkalisch | sauer |
| 4 | 4-Hydroxy-2,5,6-triamino-pyrimidin | Resorcin | rotviolett | rot | rot | hellrot |
| 5 | " | 4-Chlorresorcin | rot | rot | rot | hellrot |
| 6 | " | m-Aminophenol | rotviolett | violett | rotviolett | rot |
| 7 | " | m-Phenylen-diamin | rotviolett | rotviolett | grauviolett | hellgrauviolett |
| 8 | " | α-Naphthol | blauviolett | blauviolett | blauviolett | hellblauviolett |
| 9 | " | p-Amino-o-kresol | rotviolett | hellrotviolett | rotviolett | hellrotviolett |
| 10 | " | 2-Amino-4-hydroxy-ethylaminoanisol | blau | blau | blau | hellblau |
| 11 | " | o-Aminophenol | goldgelb | orangefarben | gelb | gelb |
| 12 | " | o-Chlor-p-phenylen-diamin | rotbraun | hellbraun | hellgoldgelb | hellgoldgelb |
| 13 | " | 1,7-Dihydroxy-naphthalin | braun | graubraun | graublau | graubraun |
| 14 | " | 3-Dimethylamino-phenol | rotviolett | violett | rotviolett | hellrotviolett |

**Patentansprüche**

1. Mittel für die oxidative Färbung von menschlichen oder tierischen Haaren mit einem Gehalt an Entwickler- und Kupplersubstanzen, dadurch gekennzeichnet, daß es als Entwicklersubstanz alleine oder in Mischung mit anderen Entwicklersubstanzen 6-OH-2,4,5-Triaminopyrimidin (4-OH-2,5,6-Triaminopyrimidin) und/oder 5-OH-2,4,6-Triaminopyrimidin enthält.

2. Mittel nach Anspruch 1, dadurch gekennzeichnet, daß es als Kupplersubstanz Resorcin, 4-Chlorresorcin, m-Aminophenol, α-Naphthol, p-Amino-4-hydroxyethylaminoanisol, o-Aminophenol, o-Chlor-p-phenylendiamin, 1,7-Dihydroxynaphthalin, 3-Dimethylaminophenol oder Gemische davon enthält.

3. Mittel nach Anspruch 2, dadurch gekennzeichnet, daß die Entwicklersubstanz in einer Konzentration von etwa 0,05 Mol mit etwa der gleichen Menge Kupplersubstanz in ihm enthalten ist.

4. Anwendung des Mittels nach einem der Ansprüche 1 bis 3 zum Färben von menschlichem Haar, dadurch gekennzeichnet, daß das Mittel vor der Aufbringung auf das zu färbende Haar mit einem Oxidationsmittel, insbesondere Wasserstoffperoxid, vermischt wird.

5. Anwendung des Mittels zum Färben von menschlichem Haar nach einem der Ansprüche 1 bis 3, dadurch gekennzeichnet, daß die Reaktion der Entwickler- und der Kupplersubstanz(en) in dem auf das zu färbende Haar aufgetragenen Färbemittel mittels des in der Umgebungsluft enthaltenen Sauerstoffs durchgeführt wird.

**Claims**

1. Composition for the oxidative dyeing of human or animal hair, containing developing and coupling substances, characterized in that it contains as developing substance alone or in admixture with additional developing substances 6-OH-2,4,5-triaminopyrimidine (4-OH-2,5,6-triaminopyrimidine) and (or) 5-OH-2,4,5-triaminopyrimidine.

2. Composition according to claim 1, containing as coupling substance resorcinol, 4-chlororesorcinol, 3-aminophenol, 1-naphthol, p-amino-4-hydroxyethylaminoanisole, 2-aminophenol, o-chloro-p-phenylenediamine, 1,7-dihydroxynaphthalene, 3-dimethyl-aminophenol or mixtures thereof.

3. Composition according to claim 2, wherein the developing substance is contained in a concentration of about 0,05 mol with about the same quantity of coupling substance.

4. Use of the composition according to one of the claims 1 to 3 for dyeing human hair, characterized in that the composition is mixed with an oxidant, preferably hydrogen peroxide, prior to its application onto the hair to be dyed.

5. Use of the composition according to one of the claims 1 to 3 for dyeing human hair, characterized in that the reaction of developing and coupling substances in the dyeing composition applied on the hair to be dyed is effected by atmospheric oxygen of the surrounding air.

**Revendications**

1. Composition pour la teinture oxidante des cheveux humains ou animals, contenant des substances de développement et de couplage, characterisé en ce que la composition contient pour substance de développement exclusivement ou en mélange comme autres substances de développement la 6-OH-2,4,5-triaminopyrimidine (4-OH-2,5,6-triaminopyrimidine) et (ou) la 5-OH-2,4,6-triaminopyrimidine.

2. Composition selon revendication 1, characterisé en ce qu'elle contient pour substance de couplage la résorcinol, 4-chlororésorcinol, 3-aminophénol, α-naphthol, p-amino hydroxyéthylaminoanisol, 2-aminophénol, o-chloro-p-phénylènediamine, 1,7-dihydroxynaphthaline, 3-diméthylaminophenol ou mélanges des substances mentionnées.

3. Composition selon revendication 2, caracterisé en ce que la concentration des substances de devéloppement et de couplage est environ 0,05 moles.

4. Utilisation de la composition selon l'une quelconque des revendications 1 à 3 pour la teinture des cheveux humains, caracterisé en ce que la composition est mélangeé comme un agent oxidant, en particulier d'eau oxygénée, avant l'application sur les cheveux humains.

5. Utilisation de la composition selon l'une quelconque des revendications 1 à 3, caracterisé en ce que la réaction des substances de développement et de couplage dans la composition teinture appliqueé sur les cheveux humains est effectueé par l'oxygène contenu dans l'air ambiant.